# EUROPÄISCHE PATENTSCHRIFT

(11) **EP 0 773 866 B1**
(45) Veröffentlichungstag und Bekanntmachung des Hinweises auf die Patenterteilung: **08.04.1998**
(21) Anmeldenummer: 95926373.2
(22) Anmeldetag: 03.08.1995
(51) Int. Cl.: B30B 11/34, A61K 9/28

(54) **VERFAHREN ZUR HERSTELLUNG VON MANTELTABLETTEN**
METHOD OR PRODUCING COATED TABLETS
PROCEDE DE FABRICATION DE COMPRIMES ENROBES

(30) Priorität: 03.08.1994 DE 4427390; 20.10.1994 DE 4437442
(43) Veröffentlichungstag der Anmeldung: 21.05.1997
(73) Patentinhaber: Voss, Gunter Meinhardt, D-86911 Diessen (DE)
(72) Erfinder: Voss, Gunter Meinhardt, D-86911 Diessen (DE)
(74) Vertreter: Münich, Wilhelm, Dr.
(86) Internationale Anmeldenummer: DE9501012
(87) Internationale Veröffentlichungsnummer: WO9604128

(56) Entgegenhaltungen:
- EP-A- 0 349 777
- EP-A- 0 590 963
- DE-B- 1 025 569

## Beschreibung

### Technisches Gebiet

Die Erfindung bezieht sich auf ein Verfahren zur Herstellung von Manteltabletten aus Tablettenkernen und Mantelgranulat unter Verwendung einer Presse, die wenigstens eine Preßkammer mit vorzugsweise einem Ober- und einem Unterstempel, und eine Einbringvorrichtung für Tablettenkerne aufweist.

### Stand der Technik

Verfahren gemäß dem Oberbegriff des Patentanspruchs 1 werden in der Regel auf sogenannten Rundpressen mit hoher Verarbeitungsgeschwindigkeit ausgeführt. Hierzu wird beispielsweise auf folgende Druckschriften verwiesen: EP-A-0 349 777, EP-A-0 590 963 oder DE-AS 1 025 569.

Bei einem druckschriftlich nicht belegten, jedoch in der pharmazeutischen Industrie üblichen Verfahren erfolgt die Herstellung von Manteltabletten in zwei Schritten:

Im ersten Arbeitsschritt werden die späteren Kerne der Manteltabletten gepreßt, im zweiten Schritt wird Tablettiergut für die untere Hälfte des Mantels vorverdichtet, der Kern auf das vorverdichtete Bett aufgebracht und, nachdem Tablettiergut für die obere Hälfte des Mantels in die Matrize eingebracht worden ist, zur Manteltablette verpreßt.

Der erste Arbeitsschritt läßt sich weiter differenzieren in eine Befüllungsphase, in der das Tablettiergut in den Füllschuh eingebracht wird, in eine Verdichtungsphase und in eine Auswurfphase, in der die fertige Tablette mit Hilfe des Unterstempels aus der Matrize entfernt wird.

Der zweite Arbeitsschritt besteht herkömmlicherweise aus einer ersten Befüllungsphase, in der das Tablettiergut für die untere Hälfte der späteren Manteltablette in die Matrize gefüllt wird, aus einer Vorverdichtungsphase, in der das eingebrachte Tablettiergut leicht vorverdichtet wird, damit der einzubringende Kern plaziert werden kann, aus einer Dosierungsphase, in der der Kern in das vorverdichtete Bett eingebracht wird, aus einer zweiten Befüllungsphase, in der die obere Hälfte des Mantels in die Matrize gefüllt wird, aus einer Verdichtungsphase zur Verpressung der Manteltablette und einer Auswurfphase, die der Entfernung der gepressten Tablette aus der Matrize mit Hilfe des Unterstempels dient.

### Darstellung der Erfindung

Der Erfindung liegt die Aufgabe zugrunde, ein Verfahren zur Herstellung von Manteltabletten anzugeben, das die Herstellung von Manteltabletten vereinfacht und bei dem insbesondere lediglich ein einziger Arbeitsschritt für die Herstellung erforderlich ist.

Eine erfindungsgemäße Lösung dieser Aufgabe ist im Patentanspruch 1 gegeben. Weiterbildungen der Erfindung sind Gegenstand der Unteransprüche.

Erfindungsgemäß bestehen die Kerne nicht aus zuvor in einem separaten Preßvorgang gefertigten Tablettenkernen, die als verfestigte Masse in das Tablettiergut eingebracht werden, sondern aus einer pastösen Zubereitung der Inhaltsstoffe in einem Dispersionsmittel, wie beispielsweise Alkohol oder Miglyol. Damit ist es möglich, vor der Verpressung des Mantelgranulats wenigstens einen pastenartigen Tablettenkern in das zu verpressende Mantelgranulat einzubringen. Anschließend erfolgt die Verpressung des Mantelgranulats und der Tablettenkerne in einem einzigen Preßschritt.

Das erfindungsgemäße Verfahren ermöglicht es insbesondere, wenigstens zwei örtlich voneinander getrennte Tablettenkerne in die Tablette derart einzubringen, daß durch das umgebende Granulat verhindert wird, daß die jeweiligen Inhaltsstoffe der Tablettenkerne untereinander chemische oder physikalische Reaktionen eingehen (Anspruch 2). Damit können u.a. Inhaltsstoffe in einer Tablette vorhanden sein, die bei einer gemeinsamen Verarbeitung unerwünschte Reaktionen eingehen würden.

Das Einbringen des bzw. der pastenartigen Tablettenkerne kann auf die verschiedensten Arten erfolgen. Bevorzugt ist es jedoch, wenn gemäß Anspruch 2 die pastöse Tablettenkern-Masse eine derartige Konsistenz und insbesondere eine derartige Viskosität aufweist, daß die Tablettenkern-Masse mittels einer Hochdruck-Einbringvorrichtung in das Mantelgranulat eingespritzt wird.

Bei dem Einbringen des bzw. der Tablettenkerne tritt jedoch folgendes Problem auf:

Durch die Zusammenfassung der beiden beim Stand der Technik getrennten Arbeitsschritte zu einem einzigen Arbeitsschritt muß die Dosierung und die Abteilung der Tablettenkerne, die beim Stand der Technik durch die getrennte Verpressung der Tablettenkerne erfolgt, während des Einbringens der Tablettenkernmasse in das Tablettiergut des Tablettenmantels erfolgen. Dies hat zur Folge, daß die pastöse Tablettenkern-Masse in genau zu dosierende Mengenportionen abgeteilt werden muß.

Deshalb ist bei einem bevorzugten Ausführungsbeispiel der Erfindung eine Dosiervorrichtung vorgesehen, die den technischen Anforderungen höchstpräziser Massenflußdosierung gerecht werden muß. Diese Dosiervorrichtung besteht bei der im Anspruch 4 beanspruchten Weiterbildung aus einem Proportionalventil.

Die hohe Dosierungsgenauigkeit der Tablettenkernmasse, die bei gepreßten Tablettenkernen durch die bei der Tablettenherstellung üblichen Parameter (Volumen der Matrize, Dichte des Tablettierguts) und Kontrollwerte (Preßdruck, Druckfestigkeit) erreicht wird, wird bei den hohen Verarbeitungsgeschwindigkeiten, die insbesondere Rundläuferpressen erreichen (60 000 Tabletten und mehr pro Stunde) gemäß Anspruch 5 durch ein piezoelektrisches Proportionalventil erreicht, das proportional zur Ansteuerung arbeitet, und das durch Einstellung von Durchflußmenge und Querschnitt eine präzise Direkteinspritzung der hochkonzentrierten Masse in das Tablettiergut ermöglicht, die mit herkömmlichen Ventilen nicht möglich wäre. Die Ansteuerung dieses piezoelektrischen Proportionalventils erfolgt bevorzugt mittels eines Rechners bzw. einer elektronischen Steuereinheit. Zusätzlich können Sensoren für Druck, Temperatur etc. vorgesehen sein, deren Ausgangssignale an den Steuerrechner für das bzw. die Proportionalventile angelegt sind.

Das erfindungsgemäß verwendete Proportionalventil kann Drücke von 30 bis 50 bar und Frequenzen von 4 kHz realisieren, wobei ein Arbeitsschritt jeweils 50 Mikro-Sekunden dauert.

Im Anspruch 6 ist eine Weiterbildung des erfindungsgemäßen Verfahrens gekennzeichnet, bei dem zunächst die Preßkammer teilweise mit Mantelgranulat gefüllt wird, anschließend der oder die pastenartigen Tablettenkerne eingebracht werden, und dann die Preßkammer vollständig mit Mantelgranulat gefüllt wird. Dabei kann das in die Preßkammer eingebrachte Mantelgranulat vor dem Einbringen des oder der Tablettenkerne vorgepreßt werden (Anspruch 7).

Diese Weiterbildung hat insbesondere den Vorteil, daß nach der Vorpressung eine - für die Außenbefilmung von Tabletten übliche - Filmsubstanz auf den oder die Tablettenkerne aufgebracht werden kann. Das Aufbringen der Filmsubstanz erfolgt beispielsweise nach der Vorpressung.

Dabei können die gleichen Substanzen wie bei der bekannten "Außenbefilmung" von Tabletten, gegebenenfalls in erhöhter Konzentration (Anspruch 9) Verwendung finden. Zum Aufbringen des oder der Filme kann jeweils eine Hochdruck-Aufspritzvorrichtung mit einem rechßergesteuerten Proportionalventil verwendet werden (Anspruch 12). Als Proportionalventile können die gleichen rechnergesteuerten piezoelektrischen Proportionalventile wie bei der Dosierung der Tablettenkerne verwendet werden.

Die erfindungsgemäß vorgeschlagene "Innenbefilmung" von Manteltabletten hat den weiteren Vorteil, daß voneinander beabstandete Tablettenkerne mit unterschiedlichen Substanzen befilmt werden können. Die Substanzen der einzelnen Filme können bestimmte, unterschiedliche Zerfallseigenschaften und/oder unterschiedliche eingekapselte Wirkstoffe und/oder unterschiedliche Formulierungen aufweisen. Hierdurch erhält man Retardformen, die bei einer Einmalgabe beispielsweise differenzierte Zerfallzeiten aufweisen. Selbstverständlich können die mit dem erfindungsgemäßen Verfahren hergestellten Manteltabletten zusätzlich oder ausschließlich auch "außenbefilmt" werden.

Mit dem erfindungsgemäßen Verfahren können auch sehr große Tabletten hergestellt werden, die - insbesondere bei der Weiterbildung gemäß Anspruch 14 verfahrensbedingt - keine lösungsbehindernden hydrophoben Schmiermittel im Tabletteninneren aufweisen und somit eine schnelle Lösungsgeschwindigkeit als übliche Tabletten entsprechender Größe besitzen. Dieser geringere Schmiermittelzusatz wird durch das - an sich bekannte - Aufbringen des Schmiermittels auf die Preßkammerwände erreicht, im Unterschied zu der bekannten direkten Beimischung des Schmiermittels in das Tablettiergut. Dadurch kann auf die CO₂ -Entwicklung, die bei herkömmlichen Brausetabletten dazu benötigt wird, die Lösungsgeschwindigkeit zu erhöhen, verzichtet werden.

Gemäß Anspruch 15 kann jedoch die CO₂-Entwicklung, die hier beispielsweise durch mindestens zwei Tablettenkerne, wovon einer aus einem üblichen Hydrogencarbonat, der andere aus einer üblichen Säure besteht, verursacht wird, als Indikator für die vollständige Auflösung des Mantels, der in diesem Fall Wirkstoffe, Geschmacksstoffe und andere Zusatzstoffe enthält, oder zur Geschmacksverbesserung verwendet werden.

Der Zusatzstoff ist beispielsweise ein adhäsiver Gelbildner, der das Aufschwimmen der CO₂-bildenden Substanz durch die bei der Aktivierung einsetzende Gasbildung verhindert.

Ausdrücklich soll klargestellt werden, daß der vorstehend verwendete Begriff "Tablette" nicht nur Tabletten im pharmazeutischen Bereich umfaßt. Unter diesem Begriff werden im Rahmen dieser Anmeldung auch "Bonbons" oder sonstige "tablettenähnliche" Gegenstände subsumiert. Im Lebensmittelbereich sind beispielsweise pastöse Kerne aus Schokolade oder Kakaobutter denkbar. Auch kömmem die Kerne gefriergetrocknete Fruchtstücke und/oder Pasten beinhalten. Ferner sind Kerne denkbar, die aus mehreren unterschiedlichen Schichten bestehen.

## Patentansprüche

1. Verfahren zur Herstellung von Manteltabletten aus Tablettenkernen und Mantelgranulat unter Verwendung einer Presse, die wenigstens eine Preßkammer mit vorzugsweise Ober- und Unterstempel, und eine Einbringvorrichtung für Tablettenkerne aufweist,
dadurch **gekennzeichnet**, daß vor der Verpressung des
Mantelgranulats wenigstens ein pastenartiger Tablettenkern in das zu verpressende Mantelgranulat eingebracht wird, und daß die Verpressung des Mantelgranulats und der Tablettenkerne in einem einzigen Preßschritt erfolgt.

2. Verfahren nach Anspruch 1,
dadurch **gekennzeichnet**, daß wenigstens zwei örtlich voneinander getrennte Tablettenkerne derart eingebracht werden, daß verhindert wird, daß deren Inhaltsstoffe chemische oder physikalische Reaktionen eingehen.

3. Verfahren nach Anspruch 1 oder 2,
dadurch **gekennzeichnet**, daß die pastöse Tablettenkern-Masse eine derartige Konsistenz und insbesondere Viskosität aufweist, daß die Tablettenkern-Masse mittels einer Hochdruck-Einbringvorrichtung in das Mantelgranulat einspritzbar ist.

4. Verfahren nach Anspruch 3,
dadurch **gekennzeichnet**, daß zum Dosieren der Tablettenkern-Masse in der Hochdruck-Einbringvorrichtung ein Proportionalventil verwendet wird.

5. Verfahren nach Anspruch 4,
dadurch **gekennzeichnet**, daß als Ventil ein rechnergesteuertes piezoelektrisches Proportionalventil verwendet wird, das proportional zur Ansteuerung arbeitet und dessen Durchflußmenge und Querschnitt einstellbar sind.

6. Verfahren nach einem der Ansprüche 1 bis 5,
dadurch **gekennzeichnet**, daß zunächst die Preßkammer teilweise mit Mantelgranulat gefüllt wird, anschließend der oder die pastenartigen Tablettenkerne eingebracht werden, und dann die Preßkammer vollständig mit Mantelgranulat gefüllt wird.

7. Verfahren nach Anspruch 6,
dadurch **gekennzeichnet**, daß das in die Preßkammer eingebrachte Mantelgranulat vor dem Einbringen des oder der Tablettenkerne vorgepreßt wird.

8. Verfahren nach Anspruch 7,
dadurch **gekennzeichnet**, daß nach der Vorpressung eine übliche Filmsubstanz auf den oder die Tablettenkerne aufgebracht werden.

9. Verfahren nach Anspruch 8,
dadurch **gekennzeichnet**, daß die Filmsubstanz verglichen mit der herkömmlichen Tablettenbefilmung in erhöhter Konzentration verwendet wird.

10. Verfahren nach Anspruch 8 oder 9,
dadurch **gekennzeichnet**, daß voneinander beabstandete Tablettenkerne mit unterschiedlichen Substanzen befilmt werden.

11. Verfahren nach Anspruch 10,
dadurch **gekennzeichnet**, daß sich die unterschiedlichen Substanzen hinsichtlich ihrer Zerfallszeit unterscheiden.

12. Verfahren nach einem der Ansprüche 8 bis 11,
dadurch **gekennzeichnet**, daß zum Aufbringen des oder der Filme jeweils eine Hochdruck-Aufspritzvorrichtung mit einem rechnergesteuerten Proportionalventil verwendet wird.

13. Verfahren nach Anspruch 12,
dadurch **gekennzeichnet**, daß als Ventil ein rechnergesteuertes piezoelektrisches Proportionalventil verwendet wird, das proportional zur Ansteuerung arbeitet und dessen Durchflußmenge und Querschnitt einstellbar sind.

14. Verfahren nach einem der Ansprüche 1 bis 13,
dadurch **gekennzeichnet**, daß zum Schnmieren des oder der Preßstempel und der Preßkammerwände eine Schmiervorrichtung mit insbesondere zwei Zweistoffdüsen verwendet werden, die das Schmiermittel auf die Preßkammerwände und insbesondere den Ober- und Unterstempel aufbringen.

15. Verwendung des Verfahrens nach einem der Ansprüche 1 bis 14 zur Herstellung von Brausetabletten, deren Mäntel Wirkstoffe, Geschmackstoffe und/oder andere Stoffe enthalten, und die mindestens zwei Tablettenkerne aufweisen, wobei einer ein übliches Hydrogencarbonat und ein anderer eine übliche Säure enthalten.

## Claims

1. Method of manufacturing coated tablets from tablet cores and coating granulate, using a press including at least one compression chamber preferably with an upper and a lower punch, and a feeding device for tablet cores,
**characterised** in that prior to compression of said coating granulate at least one pasty tablet core is introduced into the coating granulate to be compressed, and that compression of said coating granulate takes place in a single pressing step.

2. Method according to Claim 1,
**characterised** in that at least two tablet cores locally separate from one another are introduced in a way to prevent chemical or physical reactions between their ingredients.

3. Method according to Claim 1 or 2,
**characterised** in that said pasty tablet core mass has such a consistency and especially such a viscosity that the tablet core mass may be injected by means of a high-pressure feeding device into said coating granulate.

4. Method according to Claim 3,
**characterised** in that a proportional valve is used for dosing said tablet core mass in said high-pressure feeding device.

5. Method according to Claim 4,
**characterised** in that a computer-controlled piezoelectric proportional valve is used as valve, which operates proportionally to the control and whose throughput volume and cross-section are adjustable.

6. Method according to any of the Claims 1 to 5,
**characterised** in that said compression chamber is initially partially filled with coating granulate, that then the pasty tablet core(s) are introduced, and that the compression chamber is then completely filled with coating granulate.

7. Method according to Claim 6,
**characterised** in that the coating granulate introduced into said compression chamber is pre-compressed prior to introduction of the tablet core(s).

8. Method according to Claim 7,
**characterised** in that a conventional film substance is applied to said tablet core(s) after pre-compression.

9. Method according to Claim 8,
**characterised** in that compared against the conventional tablet film coating operation, said film substance presents a higher concentration.

10. Method according to Claim 8 or 9,
**characterised** in that mutually spaced tablet cores are film-coated with different substances.

11. Method according to Claim 10,
**characterised** in that the different substances differ in terms of their disintegration time.

12. Method according to any of the Claims 8 to 11,
**characterised** in that a high-pressure injection device with a computer-controlled proportional valve is used for applying said film or films.

13. Method according to Claim 12,
**characterised** in that a computer-controlled piezoelectric proportional valve is used as valve, which operates proportionally to the control and whose throughput volume and cross-section are adjustable.

14. Method according to any of the Claims 1 to 13,
**characterised** in that a lubricating device with two dual-substance nozzles in particular is used for lubricating said press punch or punches and the walls of said compression chamber, which nozzles apply the lubricant on the compression chamber walls and especially the upper and lower punches.

15. Application of the method according to any of the Claims 1 to 14 for manufacturing effervescent tablets whose coatings contain active substances, flavouring substances and/or other substances, and which include at least two tablet cores whereof one contains a conventional hydrogen carbonate whilst the other one contains a conventional acid.

## Revendications

1. Procédé de fabrication de comprimés en noyaux de comprimés et granulé d'enrobage, en utilisant une presse qui comprend au moins une chambre de compression, de préférence à une étampe supérieure et une étampe inférieure, et un dispositif chargeur à charger lesdits noyaux de comprimés,
**caractérisé** en ce qu'avant la compression dudit granulé d'enrobage on injecte au moins un noyau pâteux dans granulé d'enrobage à comprimer, et en ce que la compression dudit granulé d'enrobage se fait dans une seule opération de pressage.

2. Procédé selon la revendication 1,
**caractérisé** en ce qu'au moins deux noyaux de comprimés, qui sont espacés l'un de l'autre localement, sont introduits de façon à empêcher des réactions chimiques ou physiques entre leur substances y contenues.

3. Procédé selon la revendication 1 or 2,
**caractérisé** en ce que la pâte dudit noyau de comprimé pâteux a une telle consistance et en particulier une telle viscosité que la pâte du noyau de comprimé puisse être injectée moyennant un dispositif chargeur à haute pression dans ledit granulé d'enrobage.

4. Procédé selon la revendication 3,
**caractérisé** en ce qu'une soupape proportionnelle est utilisée à doser la pâte dudit noyau de comprimé dans ledit dispositif chargeur à haute pression.

5. Procédé selon la revendication 4,
**caractérisé** en ce qu'une soupape proportionnelle piézoélectrique commandée par ordinateur est utilisée comme la soupape, qui fonctionne de façon proportionnelle à la commande et dont le débit de passage et le profil en travers sont réglables.

6. Procédé selon une quelconque des revendications 1 à 5,
**caractérisé** en ce que ladite chambre de compression est au début remplie en partie du granulé d'enrobage, en ce qu'ensuite ledit ou lesdits noyau(x) de comprimés pâteux sont introduits, et en ce que la chambre de compression est ci-après remplie complètement du granulé d'enrobage.

7. Procédé selon la revendication 6,
**caractérisé** en ce que le granulé d'enrobage chargé dans ladite chambre de compression est précomprimé avant l'introduction du ou des noyau(x) de comprimé(s).

8. Procédé selon la revendication 7,
**caractérisé** en ce qu'une substance de laquage conventionnelle est appliquée sur ledit ou lesdits noyau(x) de comprimé(s) après la précompression.

9. Procédé selon la revendication 8,
**caractérisé** en ce que ladite substance de laquage présente une concentration plus grande qu'au cas d'une opération d'enrobage par laquage de comprimés conventionnelle.

10. Procédé selon la revendication 8 or 9,
**caractérisé** en ce que des noyaux de comprimés espacés l'un de l'autre sont enrobés par laquage par des substances différentes.

11. Procédé selon la revendication 10,
**caractérisé** en ce que les substances différentes se distinguent l'une de l'autre par leur temps de désintégration.

12. Procédé selon une quelconque des revendications 8 à 11,
**caractérisé** en ce qu'un dispositif injecteur à haute pression, à une soupape proportionnelle commandée par ordinateur est utilisé afin d'appliquer ledit film ou lesdits films.

13. Procédé selon la revendication 12,
**caractérisé** en ce qu'une soupape piézoélectrique proportionnelle commandée par ordinateur proportionnelle est utilisée comme la soupape, qui fonctionne de façon proportionnelle à la commande et dont le débit de passage et son profil en travers sont réglables.

14. Procédé selon une quelconque des revendications 1 à 13,
**caractérisé** en ce qu'un dispositif graisseur à deux fières pour deux substances, en particulier, est utilisé à lubrifier ladite étampe ou lesdites étampes de presse ainsi que les parois de ladite chambre de compression, lesquelles filières appliquent le lubrifiant sur les parois de la chambre de compression et en particulier des étampes supérieure et inférieure.

15. Emploi du procédé selon une quelconque des revendications 1 à 14 dans la fabrication des comprimés gazeux dont les enrobages contiennent des matières actives, des aromates artificiels et/ou des autres substances, et qui comprennent au moins deux noyaux de comprimés dont l'un contient un carbonate d'hydrogène conventionnel pendant que l'autre contient un acide conventionnel.
